# EUROPEAN PATENT SPECIFICATION

(11) **EP 1 359 225 B1**
(45) Date of publication and mention of the grant of the patent: **15.11.2006**
(21) Application number: 03005578.4
(22) Date of filing: 12.03.2003
(51) Int. Cl.: C12Q 1/00

(54) **Assay for synthesis activity of hydrolases**
Verfahren zur Bestimmung der Syntheseaktivität von Hydrolasen
Procédé pour la détérmination de l'activité de synthèse des hydrolases

(30) Priority: 08.04.2002 EP 02007819; 20.06.2002 EP 02013680
(43) Date of publication of application: 05.11.2003
(73) Proprietor: Degussa AG, 40474 Düsseldorf (DE)
(72) Inventor: Bornscheuer, Uwe T. Prof. Dr., 17489 Greifswald (DE); KANARZYCKA, Monika, 17489 Greifswald (DE); Hills, Geoffrey, Dr., 45355 Essen (DE)

(56) References cited:
- EP-A- 0 577 446
- WO-A-00/28007
- US-A- 4 022 667
- US-A- 4 212 939
- NISHIO T ET AL: "PRODUCTION OF OPTICALLY ACTIVE ESTERS AND ALCOHOLS FROM RACEMIC ALCOHOLS BY LIPASE-CATALYZED STEREOSELECTIVE TRANSESTERIFICATION IN NON-AQUEOUS REACTION SYSTEM" JOURNAL OF BIOCHEMISTRY (TOKYO), vol. 105, no. 4, 1989, pages 510-512, XP001095149 ISSN: 0021-924X
- SUZUKI TAKESHI ET AL: "A cold-active esterase with a substrate preference for vinyl esters from a psychrotroph, Acinetobacter sp. strain no. 6: Gene cloning, purification, and characterization." JOURNAL OF MOLECULAR CATALYSIS B ENZYMATIC, vol. 16, no. 5-6, 2002, pages 255-263, XP001094744 ISSN: 1381-1177
- PATENT ABSTRACTS OF JAPAN vol. 016, no. 061 (C-0910), 17 February 1992 (1992-02-17) & JP 03 258772 A (KAZUHIRO IMAI), 19 November 1991 (1991-11-19)

## Description

The present invention concerns a way to detect the feasibility of a special family of enzymes to produce chemical compounds. In particular the invention is directed to an assay and a method to screen libraries of hydrolases, in particular esterases, lipases, proteases, amidases and acylases for their facility to catalyse esterification, transesterification and interesterification reactions in non-aqueous, organic media.

Hydrolases, in particular lipases and esterases are versatile biocatalysts and find increasing application in organic syntheses (U.T. Bornscheuer, R. J. Kazlauskas, Hydrolases in organic synthesis - regio- and stereoselective biotransformations, Wiley-VCH, Weinheim, 1999; K. Faber, Biotransformations in Organic Chemistry, 4 ed., Springer, Berlin, 2000, K.Drauz, H. Waldmann (Ed.), Enzymes in Organic Synthesis, Wiley-VCH, Weinheim, 2002) The main interest stems for their good to excellent stereoselectivity and their exceptionally high activity and stability in organic systems. Thus, not only hydrolysis of esters can be studied, but also synthesis reactions are possible. Indeed, the majority of reactions are currently performed in organic media and are even performed on industrial scale (A. Liese et al., Industrial Biotransformations, Wiley-VCH, Weinheim, 2000; U. T. Bornscheuer, in Biotechnology, Vol. 8b (Eds.: H. J. Rehm, G. Reed, A. Pühler, P. J. W. Stadler, D. R. Kelly), Wiley-VCH, Weinheim, 2000, pp. 277-294; F. Balkenhohl et al., J. Prakt. Chem. 1997, 339, 381-384). This includes the synthesis of optically pure building blocks, flavors and fragances, lipid modification and also synthesis of simple esters such as myristyl myristate for cosmetic applications.

Identification of suitable biocatalysts is usually performed by small-scale esterification reactions, which can be monitored by GC or HPLC analysis to determine conversion and - if applicable - optically purities. Broad investigations of a large number of enzymes - especially of huge mutant libraries accessible by directed evolution (F. H. Arnold, A. A. Volkov, Curr. Opin. Chem. Biol. 1999, 3, 54-59; F. H. Arnold, Acc. Chem. Res. 1998, 31, 125-131; U.T. Bornscheuer, Angew. Chem. Int. Ed. Engl. 1998, 37, 3105-3108; M.T. Reetz, K.-E. Jaeger, Topic Curr. Chem. 1999, 200, 31-57) - and optimisation of reaction conditions (different alcohols or acyl donors, solvents, temperature variation etc.) are therefore very time-consuming.

Alternatively, active (and stereoselective) biocatalysts are usually identified by means of hydrolytic assays, for which a considerable number of useful high-throughput methods have been developed recently (M. T. Reetz, Angew. Chem. Int. Ed. Engl. 2001, 40, 284-310; D. Wahler et al., Angew. Chem. Int. Ed. Engl. 2001, 40, 4457-4460; D. Wahler, J.L. Reymond, Curr. Opin. Biotechnol. 2001 12, 535-544; M. Baumann et al., Angew. Chem. Int. Ed. Engl. 2001, 40,. 4201-4204; L. E. Janes, R. J. Kazlauskas, J. Org. Chem. 1997, 62, 4560-4561). Unfortunately, the above mentioned optimisations can not be studied in aqueous systems if a reaction in organic solvents is envisaged and it often turns out that results from hydrolysis reactions can not be transferred to synthesis in organic solvents or solvent-free systems.

The object of the present invention was, therefore, to find an assay which allows the probing of large numbers of hydrolases according to their applicability in catalysing esterification, transesterification and interesterification reactions in non-aqueous, organic systems on a high-throughput level.

By applying an one pot assay to detect the synthesis activity of hydrolases comprising:
(i) transesterification of an ester of a carboxylic acid and a vinylogous alcohol with a further alcohol or ester in the presence or absence of a non aqueous solvent and the respective enzyme,
(ii) reacting the thus produced carbonylic compound with a further molecule indifferent to the reaction under (i) whereby
(iii) an easily detectable reaction product is obtained, the artisan gains the possibility to manufacture a huge amount of analysis of such synthesis reactions within a short time period automatically and gets information not only on qualitative but also on quantitative turnovers helping to select the most appropriate enzymes and/or reaction conditions and/or substrates for the transformation in question.

As already mentioned the screening can be done automatically by robots or the like. Vessels in which hundreds of reactions can be performed and analysed in parallel are known to the skilled worker. Suitable are so called microtiter plates (MTPs) for such purpose. Also feasible is the use of other equipment known to the skilled worker for enzymatic reactions such as cuvettes, eppendorf tubes, or even agar plates.

Hydrolases are a separate class of enzymes (E.C. 3). They possess the possibility to cleave chemical bonds hydrolytically. They encompass subclasses of enzymes like esterases, lipases, proteases, amidases or acylases which also are advantageously screened by the assay of the invention.

It is a key feature of the present assay that during said transesterification reaction a carbonylic compound as reactive intermediate is produced which can be detected indirectly. The reactive intermediate has to fulfil special requirements to be advantageously applied in this assay. Firstly, it has to be released by the reaction of the hydrolase in question with the enol ester. Secondly, it shall react with a further molecule present, thus rendering the characteristics of this compound in a way to be easily detectable. Alcohols matching these requirements are those which can be released irreversibly from the acid moiety like vinylogous alcohols having a C=C-double adjacent to the alcoholic function. Upon release such alcohols undergo a keto-enol tautomerisation yielding the corresponding carbonyl compound.
The skilled worker is aware of different kinds of reagents capable of reacting in proposed ways. Most preferably vinyl alcohol or 1-propen-2-ol leading to acetaldehyde or acetone, respectively, are engaged.

The carbonylic compound, like acetaldehyde or acetone, as already mentioned in turn reacts with a further molecule rendering it easily detectable.
According to the invention the skilled worker is well aware of different kinds of easy detection methods. These have to be applied in accordance with the detectable reaction product to be measured. Appropriate methods include e.g. spectrophotometric and fluorimetric measurements (D. Wahler et al., Angew. Chem. Int. Ed. Engl. 2001, 40, 4457-4460; E. Henke, U. T. Bornscheuer, Biol. Chem. 1999, 380, 1029-1033; M. Baumann et al., Angew. Chem. Int. Ed. Engl. 2001, 40, 4201-4204; L. E. Janes, R. J. Kazlauskas, J. Org. Chem. 1997, 62, 4560-4561). Preferred are detection methods which can provide information both on qualitative and on quantitative transesterification progress. More preferred are those which are easy to handle and best implemented in automatic processing. Spectrometric detection serves well in this respect. Most preferred a fluorimetric analysis method is applied.
The further molecule to be modified has to have special features to be successfully applied in the assay of the invention. On the one hand it has to act indifferent in view of the enzymatic transesterification, neither inhibiting nor otherwise adversely effecting it. One the other hand it shall react easily and quantitatively with the carbonylic compound released by the enzymatic transesterification under the conditions employed.
Moreover, relative to the detection method applied the further molecule's characteristics are thereby changed in such a way as to be monitored with an appropriate sensitivity. Thus the selection of the further molecule is directly linked to the carbonyl compound produced and the detection method employed. Numerous reagents are known for the derivatisation of carbonyl compounds (for an overview see M. Vogel et al., Fresenius J. Anal. Chem. 2000, 366, 781-791) and are used mostly in e.g. environmental analysis. However, the derivatisation conditions used may be incompatible with enzymatic reactions and the derivative of the carbonyl compound must be separated (e.g. by HPLC) from the derivatisation reagent as their mixture can not be analyzed directly. The skilled artisan may think of special further molecules like dansyl derivatives (S. Houdier et al., Anal. Chim. Acta 1999, 382, 253-263) or perylenes (H. Langhals, W. Jona, Chem. Eur. J. 1998, 4, 2110-2116). Advantageously, in view of the release of a carbonylic compound, like acetaldehyde or acetone, the indifferent reagent is selected from the group of aromatic hydrazines. Most preferred the reagent 4-(7-nitro-2,1,3-benzoxadiazole)-hydrazine (or the derivatives as mentioned in ref. S. Uzu et al, Analyst 1990, 115, 1477-1482) is employed. The commercially available 4-(7-nitro-2,1,3-benzoxadiazole)-hydrazine (NBD-H) is a promising reagent in this respect, as it shows almost no fluorescence, whereas the corresponding hydrazone shows very high fluorescence. Moreover, the derivatisation can be performed in organic solvents at variable temperatures.

The hydrolases to be screened shall be employed for esterification, transesterification and interesterification reactions on large scale production. Inherently to these reactions is the fact that they can not be performed in aqueous solution. As already mentioned hydrolases tested for hydrolysis of esters in aqueous medium are seldom thus effective if employed in non aqueous systems. Therefore, the assay according to the invention is advantageously also performed in non-aqueous solution or without the addition of a further solvent, e.g. in neat form or in the alcohol to be transformed. Non-aqueous solvents which may be used shall have the ability to dissolve all and may not disturb the reactions between the reactants employed. Moreover, feasible media encompass those not adversely effecting the detection method applied. Preferably the solvents in which the assay is conducted are selected from the group of alkanes, cycloalkanes, aromatic hydrocarbons, ethers, tertiary alcohols or other solvents known to the artisan used as organic media in enzymatic synthesis reactions (U.T. Bornscheuer, R. J. Kazlauskas, Hydrolases in organic synthesis - regio- and stereoselective biotransformations, Wiley-VCH, Weinheim, 1999; K. Faber, Biotransformations in Organic Chemistry, 4 ed., Springer, Berlin, 2000, K.Drauz, H. Waldmann (Ed.), Enzymes in Organic Synthesis, Wiley-VCH, Weinheim, 2002). Most preferred are linear or branched pentane, hexane, heptane, toluene, octane, cyclopentane, cyclohexane, methyl cyclohexane, petrol ether, methyl-t-butyl ether, diethyl ether, diisopropylether, tertiary butanol, 2-methyl-2-butanol, DMF, DMSO, THF, methylenchloride, chloroform or mixtures thereof.

The assay according to the invention is run most properly at a temperature where the hydrolase tested are acting favourably. Thus the temperature ranges from -15°C to over 100°C for, for example, enzymes of thermophilic organisms. In usual cases the temperature applied varies from 0°C to 60°C, most preferred from 15°C to 50°C.

In a further embodiment the invention is directed to a method for detecting the synthesis activity of hydrolases using an assay according to the invention. Furthermore, the substrate specificity and stereoselectivity of a particular hydrolase enzyme or enzymes can be efficiently determined by varying the carboxylic acid and alcohol moieties of the compounds to be tested in the assay.

The assay format is in principle also applicable to the determination of enantioselectivities of special substrates. In case of chiral carboxylic acids, the corresponding vinyl esters can be used. These compounds can be easily synthesized from the chiral carboxylic acid e.g. using vinyl acetate in the presence of palladium(II)acetate (E. Henke et al., Chem. Month. 2000, 131, 633-638). For the resolution of e.g. chiral alcohols, vinyl acetate can serve as acyl donor.

One further aspect of the assay in question is its use as a probe for special carboxylic acids or alcohols in organic media if specifically acting enzymes are employed.

For an example the new assay format based on hydrolase catalysed transesterifications between vinyl laurate and 1-propanol is described. Acetaldehyde released stoichiometrically reacts *in situ* with a hydrazine producing a fluorogenic derivative, which can be quantified by fluorescence measurements (Scheme 1). 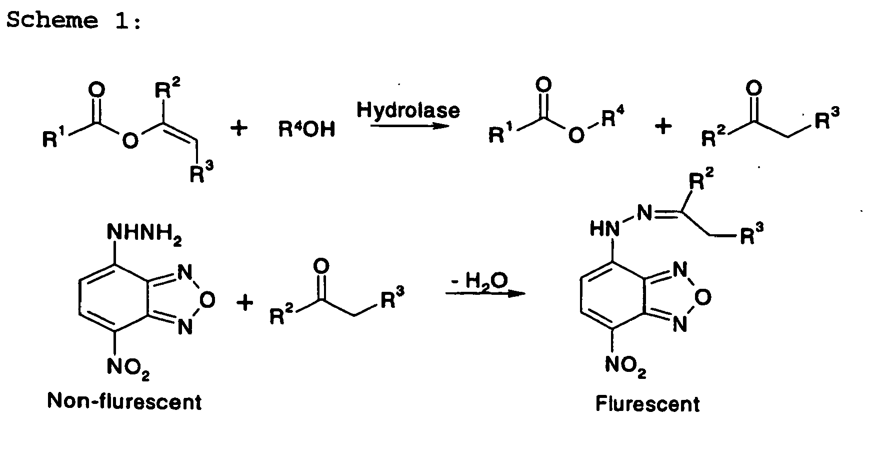 R¹ may be the rest of the carboxylic acid as defined in the text or (C₁-C₈)-Alkyl, (C₂-C₈)-Alkoxyalkyl, (C₆-C₁₈)-Aryl, (C₇-C₁₉)-Aralkyl, (C₃-C₁₈) -Heteroaryl, (C₄-C₁₉)-Heteroaralkyl, (C₁-C₈)-Alkyl- (C₆-C₁₈)-Aryl, (C₁-C₈) -Alkyl-(C₃-C₁₈)-Heteroaryl, (C₃-C₈) -Cycloalkyl, (C₁-C₈)-Alkyl-(C₃-C₈) -Cycloalkyl, (C₃-C₈)-Cycloalkyl-(C₁-C₈)-Alkyl.

R² or R³ are independently of each other H or (C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl- (C₃-C₁₈)-Heteroaryl , (C₃-C₈)-Cycloalkyl, (C₁-C₈)-Alkyl- (C₃-C₈)-Cycloalkyl, (C₃-C₈)-Cycloalkyl-(C₁-C₈) -Alkyl.

R⁴ is (C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₆)-Alkyl- (C₆-C₁₈) -Aryl, (C₁-C₆)-Alkyl- (C₃-C₁₈)-Heteroaryl, (C₃-C₈) -Cycloalkyl, (C₁-C₈)-Alkyl- (C₃-C₈)-Cycloalkyl, (C₃-C₈)-Cycloalkyl-(C₁-C₈)-Alkyl.

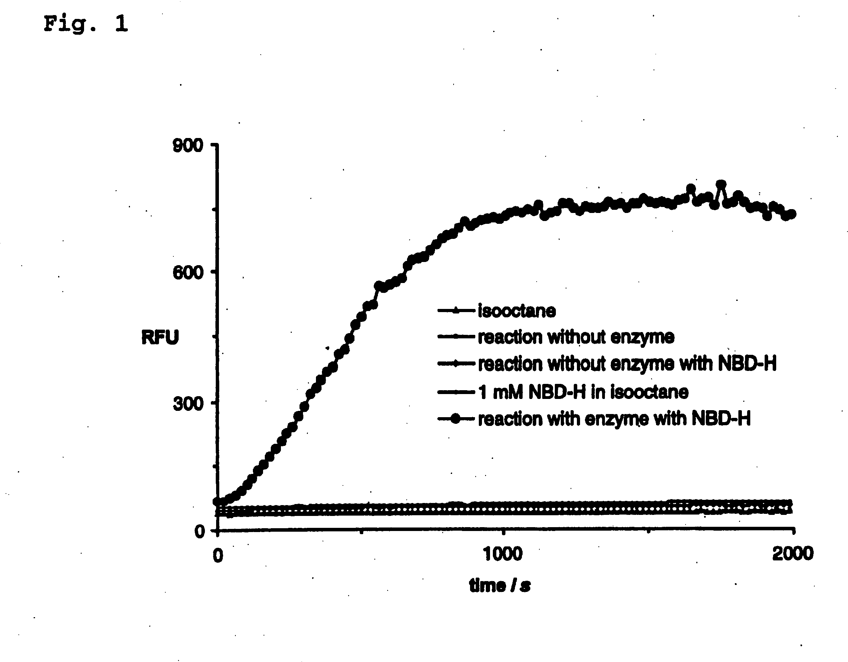

This model reaction catalysed by lipase B from *Candida antarctica* (CAL-B) was studied in greater detail. As a control, the reaction progress was monitored by GC-analysis revealing that >93% conversion can be achieved within ∼45 min in isooctane or *n*-hexane (at 1:1 ratio of substrates using 0.5 % (w/w) CAL-B). Next, the model reaction was performed in microtiter plates (MTPs) in the presence of NBD-H. Fig. 1 shows a typical time course of this reaction as monitored by fluorescence measurements. This clearly shows, that the progress of the reaction can be easily monitored. However, it is recommended to use tape-sealed MTPs to avoid evaporation of the highly volatile acetaldehyde, here measurement of fluorescence is preferentially performed from the bottom of the MTP. Moreover, in most cases a good correlation between increase in fluorescence and conversion determined by GC analysis from samples of the reaction mixture can be found. Thus, this assay format is extremely versatile for the determination of synthetic activity of hydrolases in a high-throughput format and thus enables a rapid identification of active enzymes or appropriate reaction conditions.

The variety of carboxylic acids which can be applied in the synthesis screening assay is broad. Examples are acetic acid, propionic acid etc. , acrylic acid, fatty acids like oleic acid, aromatic acids like benzoic acid, lactic acid, natural and unnatural amino acids, cinnamic acid, 2-methylbutanoic acid, 2-ethylhexanoic acid, 2-chloropropionic acid or sugar acids.

As alcohol compounds like n-alcohols, 2-ethylhexanol, 2-alcohols such as isopropanol or 2-butanol, diols such as 1,2-butandiol, polyols such as carbohydrates or glycerin or polyglycerin, polyethers such as polyethyleneglycol, phenol, halogen containing alcohols such as 2-chloroethanol, 2-chloropropanol can be used.

The compounds listed are by no means intended to be restrictive to the invention. Principally, all types of carboxylic acids and alcohols are possible within the focussed limitations.

(C₁-C₈)-Alkyl may be regarded as being methyl, ethyl, n-propyl, isopropyl, n-butyl, isobutyl, sec-butyl, tert-butyl, pentyl, hexyl, heptyl or octyl, including all isomers either saturated or unsaturated. They may be mono- or poly-substituted by (C₁-C₈)-alkoxy, (C₁-C₈)-haloalkyl, OH, halogen, NH₂, NO₂, SH, S-(C₁-C₈)-alkyl, (C₁-C₈)-acyl. The radicals just described may be mono- or poly-substituted by halogens and/or by radicals containing N, O, P, S, Si atoms. They are in particular alkyl radicals of the type mentioned above that contain one or more of those hetero atoms in their chain or that are bonded to the molecule via one of those hetero atoms.

(C₃-C₈)-Cycloalkyl is to be understood as being cyclopropyl, cyclobutyl, cyclopentyl, cyclohexyl and cycloheptyl radicals, etc. either saturated or unsaturated. They may be substituted by one or more halogens and/or radicals containing an N, O, P, S atom and/or may have in the ring radicals containing an N, O, P, S atom, such as, for example, 1-, 2-, 3-, 4-piperidyl, 1-, 2-, 3-pyrrolidinyl, 2-, 3-tetrahydrofuryl, 2-, 3-, 4-morpholinyl. They may also be mono- or poly-substituted by (C₁-C₈)-alkoxy, (C₁-C₈)-haloalkyl, OH, Cl, NH₂, NO_{2,} (C₁-C₈)-acyl.

A (C₆-C₁₈)-aryl radical is to be understood as being an aromatic radical having from 6 to 18 carbon atoms. Such radicals include especially compounds such as phenyl, naphthyl, anthryl, phenanthryl, biphenyl radicals. It may be mono- or poly-substituted by (C₁-C₈)-alkoxy, (C₁-C₈)-haloalkyl, OH, halogen, NH₂, NO₂, SH, S-(C₁-C₈)-alkyl, (C₁-C₈)-acyl

A (C₇-C₁₉)-aralkyl radical is a (C₆-C₁₈)-aryl radical bonded to the molecule *via* a (C₁-C₈)-alkyl radical.

(C₁-C₈)-Alkoxy is a (C₁-C₈)-alkyl radical bonded to the molecule in question via an oxygen atom.

(C₁-C₈) -Haloalkyl is a (C₁-C₈)-alkyl radical substituted by one or more halogen atoms.

Within the scope of the invention, a (C₃-C₁₈)-heteroaryl radical denotes a five-, six- or seven-membered aromatic ring system of from 3 to 18 carbon atoms that contains hetero atoms such as, for example, nitrogen, oxygen or sulfur in the ring. Such heteroaromatic radicals are to be regarded as being especially radicals such as 1-, 2-, 3-furyl, such as 1-, 2-, 3-pyrrolyl, 1-, 2-, 3-thienyl, 2-, 3-, 4-pyridyl, 2-, 3-, 4-, 5-, 6-, 7-indolyl, 3-, 4-, 5-pyrazolyl, 2-, 4-, 5-imidazolyl, acridinyl, quinolinyl, phenanthridinyl, 2-, 4-, 5-, 6-pyrimidinyl. It may be mono- or poly-substituted by (C₁-C₈)-alkoxy, (C₁-C₈)-haloalkyl, OH, halogen, NH₂, NO₂, SH, S-(C₁-C₈)-alkyl, (C₁-C₈)-acyl.

A (C₄-C₁₉)-heteroaralkyl is to be understood as being a heteroaromatic system corresponding to the (C₇-C₁₉)-aralkyl radical.

(C₁-C₈)-Acyl is to be understood as being a (C₁-C₈) -alkyl radical bonded to the molecule *via* a -C=O- function.

Suitable halogens are fluorine, chlorine, bromine and iodine.

Within the scope of the invention, the expression enantiomerically concentrated is to be understood as meaning the proportion of an enantiomer in admixture with its optical antipodes in a range > 50 % and < 100 %.

### Experimental

Materials: 4-hydrazino-7-nitro-2,1,3-benzoxadiazole (NBD-H) was purchased from Fluka. All other chemicals were obtained at the highest purity available from common commercial suppliers.

Fluorimetric analysis was performed with the FLUOstar *Galaxy* from BMG Labtechnologies GmbH (Offenburg, Germany) using an excitation wave-length of 485 nm, and an emission wave-length of 520 nm or 590 nm at gain 0. The total reaction volume was 200-220 µl per well.

Gas-chromatographic analysis: This was performed on a Hewlett Packard GC (5890 Series II with HP 3365 Series II ChemStation) equipped with a Permabond FFAP column (Macherey & Nagel, Düren, Germany), 25 m x 0.25 mm. Samples were analysed under the following conditions: isothermal (140°C), detection temperature: 250°C, injection temperature: 230°C. Hydrogen served as carrier gas.

Lipase-catalyzed synthesis of 1-propyl laurate: Solvent free system, 1:1 ratio (vinyl laurate:1-propanol): 260 µl (1 mmol) of vinyl laurate and 60 µl (1 mmol) of 1-propanol were combined in an Eppendorf tube (1.5 ml) and CAL-B (1-10 mg) was added. The mixture was shaken at 900 rpm at 60°C. Samples were diluted with chloroform, vortexted, centrifuged and analyzed by GC. In a similar manner, reactions at a 1:10 ratio were performed. Reactions with solvents: 77,5 µl (0.3 mmol) of vinyl laurate, 22,5 µl (0.3 mmol) of 1-propanol and 900 µl of solvent (*n*-hexane, isooctane, petroleum ether) are combined in an Eppendorf tube (1.5 ml). Reactions were performed and analyzed as described above.

Fluorescence of NBD-H: In general, 200 µl of a solution of NBD-H (0.1, 0.25, 0.5 and 1 mM) in various solvents (methanol, 1-propanol, n-hexane, isooctane, petrol ether (boiling range 100-140°C), DMSO) were transferred into a microtiter plate. Then fluorescence was measured at 20°C, 25°C or 45°C for 30-50 min at the wave-lengths indicated above. In a similar manner, derivatization of acetaldehyde was studied by addition of 10 µl acetaldehyde (0.1 nmol) dissolved in the organic solvent using a micro pump within the fluorimeter. Reaction mixtures were mixed with the internal shaker of the fluorimeter at 170 rpm.

Typical procedure for the determination of synthetic activity in a microtiter plate: A mixture (210 µl) of vinyl laurate (200 nmol), 1-propanol (2 µmol) (1:10 ratio) and NBD-H (200 nmol) in 210 µl isooctane was prepared. After addition of ∼0.02 mg CAL-B, the plates were tape-sealed and inserted into the fluorimeter and shaken at 45°C at 170 rpm. The change in fluorescence was monitored by measurement from the bottom for 20 min. As weighing of these small amounts of enzyme are very difficult to achieve reproducible results, a known amount of enzyme was dissolved in phosphate buffer and then distributed to the wells of a MTP. Next, the solution was freeze-dried to ensure an exact amount of biocatalysts per reaction. Finally, substrates, organic solvent and NDB-H were added as described above.

### Enzymatic Reaction:

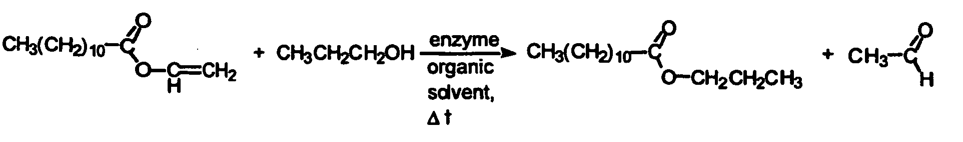

a) reactions in ratio 1: 10 in mixture DMF/TBME at 60 °C, 10% reactants in solvent, 1% amount of enzyme (CAL-B and CAL-A).

These reactions were checked in 3 different solvent systems:
A - 10% DMF in TBME
B - 5% DMF in TBME
C - 1% DMF in TBME

### Method:

25,6 µl vinyl laurate and 74,3 µl 1-propanol and 900 µl solvents (A, B or C) are combined in an Eppendorf tube (1.5 ml). After 20 µl of the mixture were taken as a standard, 10 mg of enzyme is added.

The reaction is shaken at 900 rpm at 60°C. During the reaction samples are taken in regular time intervals.

Samples of 70µl of organic phase are taken from each reaction and added to 150 -200µl solvent (chloroform), vortex and centrifuged (2 min, 13000 rpm). The organic phase is transferred to a new vial and analysed by GC.

Samples were taken every 30 minutes during 2 h.

### Results:

CAL-A, system A
no conversion at all
CAL-B, system A; CAL-B, system B; CAL-B, system C

Reactions with 1% of CAL-B are very fast. After 30 minutes conversion is already higher than 98%.

### Experimental procedure for the assay

10% of substrates in reaction mixture, 0,2% enzyme (weight percent)
vinyl laurate

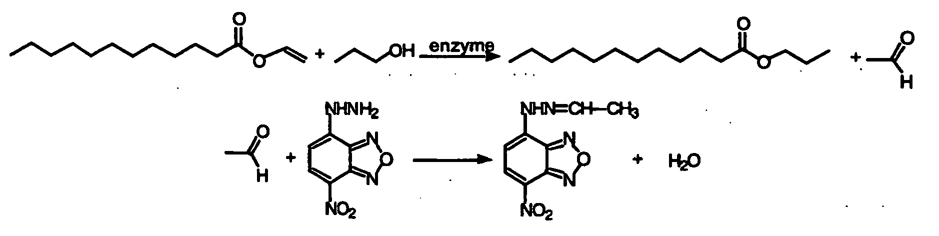

For enzymatic reactions ten different non-immobilized enzymes were selected: Chirazyme L5 (CAL-A), Chirazyme L2 (CAL-B), Amano PS (PCL), Chirazyme L9 (RML), Amano A (ANL), Amano AK (PFL), Amano AY (CRL), Chirazyme L6 (PSL), Chirazyme L3 (CRL) and SDE.

Solutions of each enzyme in sodium phosphate buffer (pH 7.5, 50 mM NaH₂PO₄) were prepared. Certain volumes of enzymatic solutions were then transferred into MTP (to get 0.22 mg of an enzyme in each well what is 1 weight% of substrates or 0.1 weight% of reaction mixture) and freeze-dried afterwards.

200 µl of a 1 mM NBD-H solution (200 nmol) were transferred into a MTP in separate wells, which contained the enzymes. Each solution was used four times. 22 µl of the reaction mixture (21,7 µmol of vinyl laurate and 217 µmol of 1-propanol) were added from the pump to each 1 mM NBD-H solution with enzyme. After addition, the MTP was tape-sealed and inserted into the fluorimeter and shaken at 45°C at 170 rpm. Fluorescence was measured in intervals of 94 s during 15 min. 1 mM NBD-H in isooctane, reaction mixture without enzyme in isooctane, reaction mixture without enzyme in 1 mM NBD-H in isooctane and each 1 mM NBD-H with enzyme suspension served as blanks (four controls each).

Fluorescence was measured from the top.

Results are taken from fluorescence analysis:

### Blanks:

A1-A4 - isooctane
A5-A8 - 1 mM NBD-H in isooctane
A9-A12 - reaction mixture in isooctane
B1-B4 - reaction mixture in 1 mM NBD-H in isooctane
B5-B8 - 1 mM NBD-H in isooctane + Amano PS
B9-B12 - 1 mM NBD-H in isooctane + Amano AK
C1-C4 - 1 mM NBD-H in isooctane + Chirazyme L6
C5-C8 - 1 mM NBD-H in isooctane + Chirazyme L3
C9-C12 - 1 mM NBD-H in isooctane + Chirazyme L2
D1-D4 - 1 mM NBD-H in isooctane + SDE
D5-D8 - 1 mM NBD-H in isooctane + Amano AY
D9-D12 - 1mM NBD-H in isooctane + Chirazyme L5
E1-E4 - 1 mM NBD-H in isooctane + Amano A
E5-E8 - 1 mM NBD-H in isooctane + Chirazyme L9

### Reactions:

E9-E12 - with Amano PS
F1-F4 - with Amano AK
F5-F8 - with Chirazyme L6
F9-F12 - with Chirazyme L3
G₁-G₄ - with Chirazyme L2
G5-G8 - with SDE
G9-G12 - with Amano AY
H1-H4 - with Chirazyme L5
H5-H8 - with Amano A
H9-H12 - with Chirazyme L9

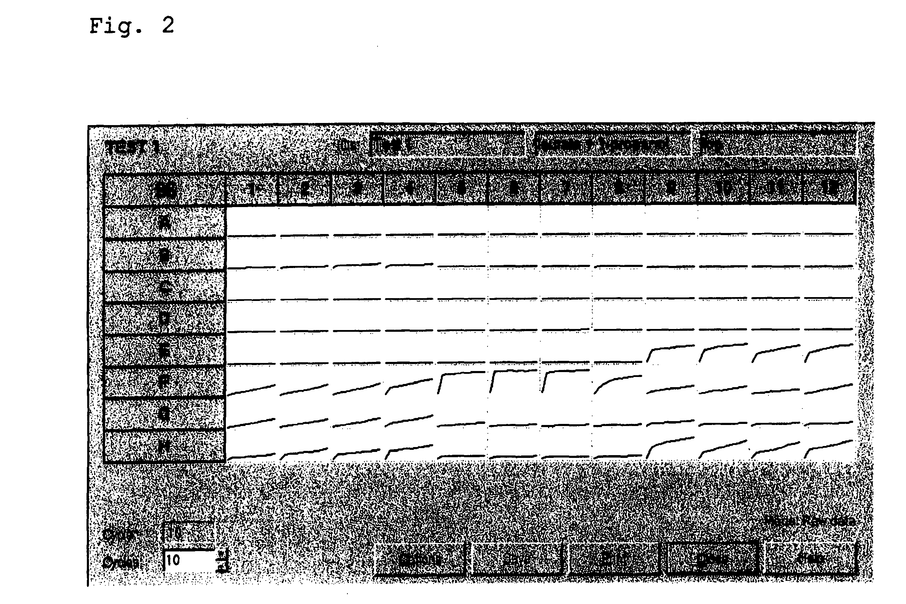

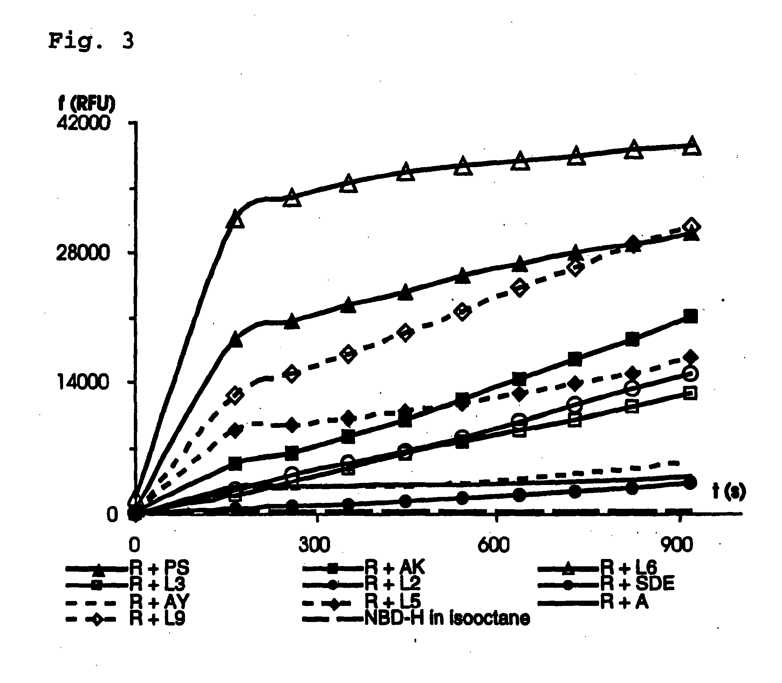

Results see Fig. 2 and 3, respectively.
R - reaction (combined enzymatic and derivatization reactions) on the MTP
PS - Amano PS = *Pseudomonas cepacia* lipase
AK - Amano AK = *Pseudomonas fluorescens* lipase
L6 - Chirazyme L6 = *Pseudomonas* species lipase
L3 - Chirazyme L3 = *Candida rugosa* lipase
L2 - Chirazyme L2 = *Candida antarctica* lipase B
SDE = *Streptomyces diastatochromogenes* esterase
AY - Amano AY = *Candida rugosa* lipase
L5 - Chirazyme L5 = *Candida antarctica* lipase A
A - Amano A = *Aspergillus niger* lipase
L9 - Chirazyme L9 = *Rhizomucor miehei* lipase

Chirazyme enzymes were purchased from Roche Diagnostics, Penzberg, Germany; Amano enzymes were purchased from Amano, Nagoya, Japan; SDE was produced by expression in E.coli according to a published procedure (Khalameyzer, V. and Bornscheuer, U.T. (1999), *Biotechnol. Lett.* 21, 101-104)

## Claims

1. Assay to detect the synthesis activity of hydrolases comprising:
(i) transesterification of an ester of a carboxylic acid and a vinylogous alcohol with a further alcohol or ester in non-aqueous media, in the presence or absence of a non-aqueous solvent, and the respective enzyme,
(ii) reacting the thus produced carbonylic compound with a further molecule indifferent to the reaction under (i) whereby
(iii) an easily detectable reaction product is obtained.

2. Assay according to claim 1,
**characterised by that** the hydrolases are esterases, lipases, proteases, amidases or acylases.

3. Assay according to claim 1 and/or 2,
**characterised in that,**
the α,β-unsatured moiety of the ester stems from vinyl alcohol or 1-propen-2-ol.

4. Assay according to any one of the preceding claims,
**characterised in that,**
the indifferent reagent is selected from the group of aromatic hydrazines.

5. Assay according to claim 4,
**characterised in that,**
the reagent is 4-(7-nitro-2,1,3-benzoxadiazole)-hydrazine.

6. Assay according to any one of the preceding claims,
**characterised in that,**
the organic solvent is selected from the group of alkanes or cycloalkanes, aromatic hydrocarbons, ethers, teriary alcohols.

7. Assay according to any one of the preceding claims,
**characterised in that,**
the temperature applied varies from -15°C to 100°C, preferably 0°C to 60°C, more preferably 15°C to 50°C.

8. Method for detecting the synthesis activity of hydrolases using a synthesis assay according to any one of the preceding claims.

9. Method for determining the substrate specificity of hydrolases using a synthesis assay according to any one of the preceding claims.

10. Method for determining the stereoselectivity of hydrolases using a synthesis assay according to any one of the preceding claims.

11. Method for determining an alcohol or carboxylic acid compound using a synthesis assay according to any one of the preceding claims in connection with a alcohol- or carboxylic acid-specific hydrolase.

12. Method for determining the enantioselectivity of chiral alcohols and chiral carboxylic acids using a synthesis assay according to any one of the preceding claims.

## Patentansprüche

1. Test zum Nachweis der Syntheseaktivität von Hydrolasen, bei dem man:
(i) einen Ester einer Carbonsäure und eines vinylogen Alkohols mit einem weiteren Alkohol oder Ester in nichtwäßrigen Medien in Gegenwart oder Abwesenheit eines nichtwäßrigen Lösungsmittels sowie des entsprechenden Enzyms transesterifiziert,
(ii) die so gebildete Carbonylverbindung mit einem weiteren, gegenüber der Umsetzung unter (i) indifferenten Molekül umsetzt, wodurch man
(iii) ein leicht nachweisbares Reaktionsprodukt erhält.

2. Test nach Anspruch 1,
**dadurch gekennzeichnet, daß**
es sich bei den Hydrolasen um Esterasen, Lipasen, Proteasen, Amidasen oder Acylasen handelt.

3. Test nach Anspruch 1 und/oder 2,
**dadurch gekennzeichnet, daß**
die α,β-ungesättigte Gruppierung des Esters aus Vinylalkohol oder 1-Propen-2-ol stammt.

4. Test nach einem der vorhergehenden Ansprüche,
**dadurch gekennzeichnet, daß**
das indifferente Reagens ausgewählt ist aus der Gruppe der aromatischen Hydrazine.

5. Test nach Anspruch 4,
**dadurch gekennzeichnet,**
**daß** es sich bei dem Reagens um 4-(7-Nitro-2,1,3-benzoxadiazol)hydrazin handelt.

6. Test nach einem der vorhergehenden Ansprüche,
**dadurch gekennzeichnet, daß**
das organische Lösungsmittel ausgewählt ist aus der Gruppe der Alkane oder Cycloalkane, aromatischen Kohlenwasserstoffe, Ether, tertiären Alkohole.

7. Test nach einem der vorhergehenden Ansprüche,
**dadurch gekennzeichnet, daß**
die angewandte Temperatur von -15 °C bis 100 °C, vorzugsweise von 0 °C bis 60 °C, stärker bevorzugt von 15 °C bis 50 °C variiert.

8. Verfahren zum Nachweis der Syntheseaktivität von Hydrolasen unter Verwendung eines Synthesetests nach einem der vorhergehenden Ansprüche.

9. Verfahren zur Bestimmung der Substratspezifität von Hydrolasen unter Verwendung eines Synthesetests nach einem der vorhergehenden Ansprüche.

10. Verfahren zur Bestimmung der Stereoselektivität von Hydrolasen unter Verwendung eines Synthesetests nach einem der vorhergehenden Ansprüche.

11. Verfahren zur Bestimmung einer Alkohol- oder Carbonsäureverbindung unter Verwendung eines Synthesetests nach einem der vorhergehenden Ansprüche in Verbindung mit einer alkohol- oder carbonsäurespezifischen Hydrolase.

12. Verfahren zur Bestimmung der Enantioselektivität chiraler Alkohole und chiraler Carbonsäuren unter Verwendung eines Synthesetests nach einem der vorhergehenden Ansprüche.

## Revendications

1. Dosage pour détecter l'activité de synthèse d'hydrolases comprenant :
(i) la transestérification d'un ester d'un acide carboxylique et d'un alcool vinylogue avec un alcool ou un ester supplémentaire dans un milieu non aqueux, en présence ou en l'absence d'un solvant non aqueux, et l'enzyme respective,
(ii) la réaction du composé carbonylique ainsi produit avec une molécule supplémentaire indifférente à la réaction en (i), ce par quoi
(iii) un produit de réaction facilement détectable est obtenu.

2. Dosage selon la revendication 1, **caractérisé en ce que** les hydrolases sont des estérases, des lipases, des protéases, des amidases ou des acylases.

3. Dosage selon la revendication 1 et/ou 2, **caractérisé en ce que** le fragment α,β-insaturé de l'ester provient de l'alcool vinylique ou du 1-propén-2-ol.

4. Dosage selon l'une quelconque des revendications précédentes, **caractérisé en ce que** le réactif indifférent est choisi dans le groupe constitué des hydrazines aromatiques.

5. Dosage selon la revendication 4, **caractérisé en ce que** le réactif est la 4-(7-nitro-2,1,3-benzoxadiazole)-hydrazine.

6. Dosage selon l'une quelconque des revendications précédentes, **caractérisé en ce que** le solvant organique est choisi dans le groupe constitué des alcanes ou des cycloalcanes, des hydrocarbures aromatiques, des éthers, des alcools tertiaires.

7. Dosage selon l'une quelconque des revendications précédentes, **caractérisé en ce** la température appliquée varie de -15°C à 100°C, de préférence de 0°C à 60°C, plus préférablement de 15°C à 50°C.

8. Procédé de détection de l'activité de synthèse d'hydrolases en utilisant un dosage de synthèse selon l'une quelconque des revendications précédentes.

9. Procédé de détermination de la spécificité de substrat d'hydrolases en utilisant un dosage de synthèse selon l'une quelconque des revendications précédentes.

10. Procédé de détermination de la stéréosélectivité d'hydrolases en utilisant un dosage de synthèse selon l'une quelconque des revendications précédentes.

11. Procédé de détermination d'un composé alcool ou acide carboxylique en utilisant un dosage de synthèse selon l'une quelconque des revendications précédentes, en rapport avec une hydrolase spécifique d'un alcool ou d'un acide carboxylique.

12. Procédé de détermination de l'énantiosélectivité d'alcools chiraux ou d'acides carboxyliques chiraux en utilisant un dosage de synthèse selon l'une quelconque des revendications précédentes.
